# EUROPÄISCHE PATENTSCHRIFT

(11) **EP 3 381 487 B1**
(45) Veröffentlichungstag und Bekanntmachung des Hinweises auf die Patenterteilung: **04.05.2022**
(21) Anmeldenummer: 18161304.3
(22) Anmeldetag: 12.03.2018
(51) Int. Cl.: A61M 5/14

(54) **EINSTECHTEIL FÜR EIN MEDIZINISCHES INFUSIONSSYSTEM**
INSERT PIECE FOR A MEDICAL INFUSION SYSTEM
ÉLÉMENT PIQUEUR POUR UN SYSTÈME DE PERFUSION MÉDICALE

(30) Priorität: 28.03.2017 DE 102017205250
(43) Veröffentlichungstag der Anmeldung: 03.10.2018
(73) Patentinhaber: B. Braun Melsungen AG, 34212 Melsungen (DE)
(72) Erfinder: Fuchs, Jürgen, 34308 Bad Emstal (DE); Schlitt, Christof, 34621 Obergrenzebach (DE); Vial, Siegbert, 35099 Burgwald (DE)
(74) Vertreter: Patentanwälte Ruff, Wilhelm, Beier, Dauster & Partner mbB

(56) Entgegenhaltungen:
- EP-A1- 1 747 787
- US-A- 4 055 176
- US-A1- 2011 275 988

## Beschreibung

Die Erfindung betrifft ein Einstechteil für ein medizinisches Infusionssystem mit einem einen Einstechdorn aufweisenden Gehäuse, das einen Fluidkanal sowie einen Belüftungskanal aufweist, der einen zu dem Fluidkanal zumindest weitgehend parallel verlaufenden ersten Kanalabschnitt sowie einen zu einer Außenseite des Gehäuses hin umgelenkten zweiten Kanalabschnitt aufweist, dem ein Luftfilterelement sowie ein Fluidabsperrglied zugeordnet sind. Die Erfindung betrifft zudem eine Tropfkammer für ein medizinisches Infusionssystem mit einem derartigen Einstechteil.

Ein derartiges Einstechteil ist aus der EP 0 582 038 B1 und in ähnlicher Form auch aus der EP 1 747 787 A1 bekannt. Das bekannte Einstechteil ist

Teil einer Tropfkammer eines medizinischen Infusionssystems. Das Einstechteil weist ein Gehäuse auf, das einen Einstechdorn umfasst. Im Bereich des Einstechdorns verlaufen ein Fluidkanal sowie ein parallel zu dem Fluidkanal erstreckter erster Kanalabschnitt eines Belüftungskanals. Der erste Kanalabschnitt geht mittels einer Umlenkung in einen zweiten Kanalabschnitt über, der zu einer Außenseite des Gehäuses hin offen ist. Der zweite Kanalabschnitt ist um 90° gegenüber dem ersten Kanalabschnitt umgelenkt. In dem zur Außenseite hin offenen zweiten Kanalabschnitt sind ein Luftfilterelement sowie ein Fluidabsperrglied in Form eines Rückschlagventils integriert. Das Gehäuse des Einstechteils ist einteilig in einem Kunststoffspritzgussverfahren hergestellt. Zur Erzielung des zweiten Kanalabschnitts, der um 90° gegenüber dem ersten Kanalabschnitt umgelenkt ist, ist im Spritzgusswerkzeug der Einsatz eines Schiebers notwendig.

Aufgabe der Erfindung ist es, ein Einstechteil und eine Tropfkammer sowie ein Spritzgusswerkzeug der eingangs genannten Art zu schaffen, die eine vereinfachte Herstellung ermöglichen.

Diese Aufgabe wird für das Einstechteil durch die Merkmale des Anspruchs 1 gelöst. Dadurch, dass der zweite Kanalabschnitt gegenüber dem ersten Kanalabschnitt nicht - wie beim Stand der Technik - quer, sondern vielmehr parallel zu dem ersten Kanalabschnitt ausgerichtet ist, ist es möglich, das Gehäuse bei einer Herstellung in einem Spritzgusswerkzeug zu entformen, ohne Schieber einzusetzen. In funktionsbereitem Zustand des Einstechteils sind demzufolge sowohl der Einstechdorn als auch der zweite Kanalabschnitt des Belüftungskanals vertikal ausgerichtet. Für die Herstellung des Gehäuses kann demzufolge ein Spritzgusswerkzeug zwei Werkzeughälften aufweisen, die in einfacher Weise entgegengerichtet zueinander entformbar sind. Eine Spritzgussform des Spritzgusswerkzeugs weist demzufolge nur eine einzige Entformungsrichtung auf. Das zusätzliche, separat hergestellte Verschlussteil gewährleistet für den funktionsbereiten Zustand des Einstechteils, dass der zweite Kanalabschnitt zu einer Innenseite des Gehäuses hin verschlossen ist. Das Verschlussteil bewirkt vorzugsweise eine 180°-Umlenkung. Erst die Montage des Verschlussteils schafft die Funktionsfähigkeit des zweiten Kanalabschnitts des Belüftungskanals. Die Herstellung des erfindungsgemäßen Einstechteils in einem Spritzgussverfahren ist erheblich wirtschaftlicher als die Herstellung eines bekannten Einstechteils. In vorteilhafter Weise ist das Einstechteil Bestandteil einer Tropfkammer eines medizinischen Infusionssystems, wobei das Gehäuse des Einstechteils vorzugsweise einen oberen Endbereich der Tropfkammer bildet. Das Gehäuse ist vorteilhaft fest mit einem transparenten Hohlzylinder der Tropfkammer verbunden.

Erfindungsgemäß weist der zweite Kanalabschnitt einen Kanalausgang zu der Außenseite des Gehäuses auf, der parallel ausgerichtet ist zu dem ersten Kanalabschnitt des Belüftungskanals. Vorteilhaft ist der gesamte zweite Kanalabschnitt einschließlich seines Kanalausgangs parallel zu dem ersten Kanalabschnitt, vorzugsweise gegenüber einer Mittellängsachse des Gehäuses zu dem ersten Kanalabschnitt seitlich nach außen versetzt, erstreckt. Das separat hergestellte Verschlussteil stellt vorteilhaft eine 180°-Umlenkung einer Luft- oder Fluidströmung für den zweiten Kanalabschnitt her.

In Ausgestaltung der Erfindung bildet das Verschlussteil eine 180°-Umlenkung zwischen dem ersten und dem zweiten Kanalabschnitt. Das Verschlussteil definiert eine Wandung des zweiten Kanalabschnitts, durch die sich eine entsprechende Strömungsumlenkung eines Luftstroms oder eines Fluidstroms zwischen den beiden zueinander parallelen Kanalabschnitten des Belüftungskanals ergibt.

In weiterer Ausgestaltung der Erfindung ist das Verschlussteil als kraftschlüssig in einem Sitz des Gehäuses positionierter Stopfen gestaltet. Der Stopfen ist zu einer Innenseite des Gehäuses geschlossen und bildet eine Wandung des zweiten Kanalabschnittes, so dass der Stopfen den zweiten Kanalabschnitt gegenüber der Innenseite des Gehäuses abdichtet.

In weiterer Ausgestaltung der Erfindung ist das Gehäuse einschließlich des Einstechdorns und der Kanalabschnitte des Belüftungskanals einteilig aus einem thermoplastischen Kunststoffmaterial in einem Spritzgussverfahren hergestellt. Eine entsprechende Spritzgussform zur Herstellung des Gehäuses weist lediglich eine einzige Entformungsrichtung auf.

Die der Erfindung zugrunde liegende Aufgabe wird zudem für die Tropfkammer durch die Merkmale des Patentanspruchs 5 gelöst.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt eine Ausführungsform einer erfindungsgemäßen Tropfkammer mit einer Ausführungsform eines erfindungsgemäßen Einstechteils im Bereich einer Oberseite der Tropfkammer,
- Fig. 2: in vergrößerter Darstellung eine Ansicht des Einstechteils gemäß Fig. 1 von unten und
- Fig. 3: in vergrößerter Darstellung einen Längsschnitt durch das Einstechteil nach Fig. 2.

Eine Tropfkammer 1 eines medizinischen Infusionssystems ist in einem Funktionszustand vertikal ausgerichtet und weist im Bereich ihrer Oberseite ein Einstechteil 2 auf. Im Bereich einer Unterseite ist ein Anschlussbereich 3 zu einem Schlauchleitungssystem des medizinischen Infusionssystems vorgesehen. Das Einstechteil 2 weist ein Gehäuse 4 auf, das einteilig aus einem thermoplastischen Kunststoffmaterial in einem Spritzgussverfahren hergestellt ist. Das Gehäuse 4 ist im Bereich seiner Unterseite mittels eines Bunds auf einen hohlzylindrischen Stirnendbereich der Tropfkammer 1 aufgesetzt und insbesondere durch Verklebung oder durch Verschweißung fest mit diesem verbunden.

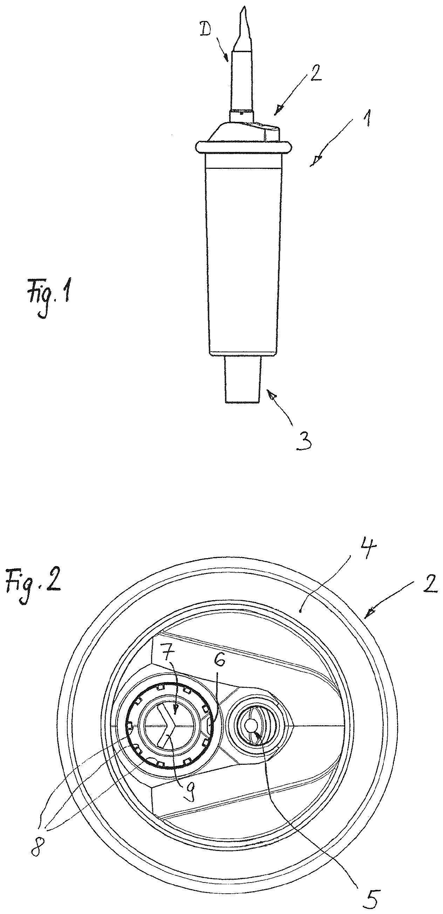

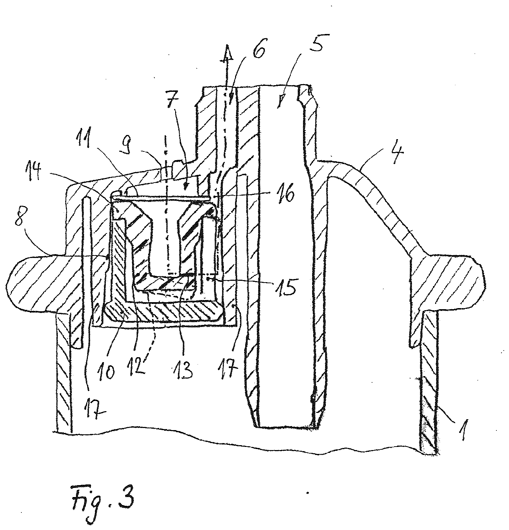

Das Einstechteil 2 weist einen Einstechdorn D auf, der gemäß den Fig. 2 und 3 mit einem Fluidkanal 5 sowie mit einem Belüftungskanal 6, 7 versehen ist. Sowohl der Fluidkanal 5 als auch der Belüftungskanal 6, 7 sind einstückige Bestandteile des Gehäuses 4 des Einstechteils 2. Auch der Einstechdorn D ist einstückiger Bestandteil des Gehäuses 4. Der Fluidkanal 5 erstreckt sich in vertikaler Ausrichtung des Einstechdorns D ebenfalls vertikal. Der Fluidkanal 5 ist zu einer Spitze des Einstechdorns D und zu einer Innenseite der Tropfkammer 1 hin offen.

Parallel neben dem Fluidkanal 5 erstreckt sich ein erster Kanalabschnitt 6 des Belüftungskanals 6, 7 in dem Einstechdorn D. Der erste Kanalabschnitt 6 ist ebenfalls - bei vertikaler Ausrichtung des Einstechdorns D - vertikal ausgerichtet und im Bereich einer Spitze des Einstechdorns D offen. Der erste Kanalabschnitt 6 ist zudem zu einem zweiten Kanalabschnitt 7 des Belüftungskanals hin offen, der parallel neben dem ersten Kanalabschnitt 6 unterhalb des Einstechdorns D im Gehäuse 4 positioniert ist. Der erste Kanalabschnitt 6 des Belüftungskanals geht an einem Übergang des Einstechdorns D in einen erweiterten Gehäuseabschnitt des Gehäuses 4 offen in den zweiten Kanalabschnitt 7 über, wobei der zweite Kanalabschnitt 7 einen wesentlich größeren freien Querschnitt aufweist als der erste Kanalabschnitt 6. Der zweite Kanalabschnitt 7 weist eine zur Innenseite des Gehäuses 4 hin offene, topfartige Form auf, die durch eine umlaufende, zylindrische Wandung 17 begrenzt ist. Der zweite Kanalabschnitt 7 ist in vertikaler Ausrichtung des Einstechdorns D ebenfalls vertikal ausgerichtet, und zwar seitlich versetzt zu dem ersten Kanalabschnitt 6. Der zweite Kanalabschnitt 7 ist im Bereich seiner Oberseite mit einem Kanalausgang 9 zur Außenseite des Gehäuses 4 hin versehen, der pfeilartig gestaltet ist (Fig. 2). Die umlaufende zylindrische Wandung 17, die den zweiten Kanalabschnitt 7 radial begrenzt, ist radial innenseitig mit Stützrippen 8 versehen, die über den Umfang der zylindrischen Wandung 17 hin verteilt angeordnet sind.

Der zweite Kanalabschnitt 7 weist im Bereich einer Wandung des Gehäuses 4 einen nicht näher bezeichneten, umlaufenden Stützbund auf, der den Kanalausgang 9 innenseitig umgibt, und der zur Stützung eines scheibenförmigen Luftfilterelements 11 vorgesehen ist.

Innenseitig an das Luftfilterelement 11 schließt ein glockenförmiger Membrankörper 12 an, der aus einem Elastomermaterial hergestellt ist. Der Membrankörper 12 weist im Bereich seiner Oberseite (auf die Darstellung in Fig. 3 bezogen) einen umlaufenden Stützbund 14 auf, der gegenüber einer becherartigen Wandung des glockenförmigen Membrankörpers 12 radial nach außen erstreckt ist. Dieser Stützbund 14 wird durch ein Verschlussteil in Form eines Stopfens 10 gegen das Luftfilterelement 11 im Bereich des Ringbunds des Gehäuses 4 gepresst. Der Stopfen 10 bildet einen Deckel zum Abschließen und Abdichten des Kanalabschnitts 7 im Bereich der Innenwandung der gehäuseseitigen zylindrischen Wandung 17. Der Stopfen 10 weist eine umlaufende Ringwandung auf, die kraftschlüssig an den Stützrippen 8 geklemmt ist. Die umlaufende Ringwandung des Stopfens 10 ist mit einem über eine gesamte Höhe der Ringwandung des Stopfens 10 radial offenen Schlitz 15 versehen, um eine Öffnung des zweiten Kanalabschnitts 7 zum ersten Kanalabschnitt 6 zu bewirken.

Der Stopfen 10 ist derart gestaltet, dass er den zweiten Kanalabschnitt 7 zur Innenseite des Gehäuses hin vollständig abdichtet. Hierzu weist der Deckel des Stopfens 10 einen umlaufenden Randbereich auf, der sich kraftschlüssig und dicht an der umlaufenden zylindrischen Wandung 17 des Gehäuses 4 abstützt. Ergänzend kann der Stopfen 10 zusätzlich zu der kraftschlüssigen Dichtwirkung noch stoffschlüssig mit der Wandung 17 verbunden sein, insbesondere durch Verklebung oder durch Verschweißung.

Der als Rückschlagventil dienende, glockenförmige Membrankörper 12 weist im Bereich seiner Unterseite einen geschlossenen Boden auf, der für eine untere Hälfte des glockenförmigen Membrankörpers 12 eine Becherform definiert. Der geschlossene Boden ist mittels eines Schlitzes 13, der in einer Radialebene seitlich oberhalb des Bodens in dem Membrankörper 12 vorgesehen ist, druckabhängig nach unten abklappbar, wodurch ein Durchtritt aus dem Inneren des Membrankörpers 12 zu dem Radialschlitz 15 hin freigegeben wird (in der Zeichnung nach rechts). Der Schlitz 13 erstreckt sich horizontal über einen Großteil des Umfangs des Membrankörpers 12, so dass der nicht geschlitzte, verbleibende Wandbereich des Membrankörpers 12 ein Scharnier in Form eines Festkörpergelenks bildet. In gestrichelter Darstellung ist eine Öffnungsstellung des Bodens gezeigt. Der Schlitz 13 ermöglicht eine Verschwenkung des elastisch verformbaren Bodens des Membrankörpers 12 in geschlossener Stellung zu einer Innenseite des Deckels des Stopfens 10 hin, wobei der Boden des Membrankörpers 12 zu der Innenseite des Deckels des Stopfens 10 beabstandet ist. Dadurch ist es möglich, im Betrieb des Einstechteils 2, in dem der Einstechdorn D in eine Unterseite eines Fluidspeicherbehältnisses eingesteckt ist, einen Luftstrom über entsprechende Druckunterschiede zwischen einem Inneren des Fluidspeicherbehältnisses und einer Umgebung gemäß der strichpunktierten Pfeildarstellung in Fig. 3 von der Außenseite des Gehäuses 4 her durch den Kanalausgang 9, durch das Luftfilterelement 11 und durch den Schlitz 13 radial zum Boden des Membrankörpers 12 hindurch zu erzielen, wobei der strichpunktiert dargestellte Luftstrom anschließend über die Innenseite des Deckels des Stopfens 10 zu dem Radialschlitz 15 hin umgelenkt und durch diesen hindurch um 180° gedreht entlang der Innenseite der Ringwandung 17 zu dem ersten Kanalabschnitt 6 nach oben geführt wird. Dadurch ist eine gewünschte Belüftung des Fluidspeicherbehältnisses ermöglicht, so dass Fluid aus dem Fluidspeicherbehältnis durch den Fluidkanal 5 im Einstechdorn D in die Tropfkammer 1 gelangen kann, ohne dass Druckunterschiede wegen mangelnder Belüftung des Fluidspeicherbehältnisses zu einem Stocken des Fluidstroms führen.

Sollte Fluid unbeabsichtigt auch über den ersten Kanalabschnitt 6 des Belüftungskanals 6, 7 aus dem Fluidspeicherbehältnis in Richtung des zweiten Kanalabschnitts 7 gelangen, drückt eine entsprechende Fluidströmung in entgegengesetzter Richtung von unten her auf eine Außenseite des Bodens des Membrankörpers 12. Der Schlitz 13 ist derart gestaltet, dass in Verbindung mit der entsprechend gestaltete elastischen Verformbarkeit des Membrankörpers 12 der Schlitz 13 bei einem solchen Fluiddruck von der Unterseite des Membrankörpers 12 her verschlossen bleibt.

## Patentansprüche

1. Einstechteil für ein medizinisches Infusionssystem mit einem einen Einstechdorn (D) aufweisenden Gehäuse (4), das einen Fluidkanal (5) sowie einen Belüftungskanal (6, 7) aufweist, der einen zu dem Fluidkanal (5) zumindest weitgehend parallel verlaufenden ersten Kanalabschnitt (6) sowie einen zu einer Außenseite des Gehäuses (4) hin umgelenkten zweiten Kanalabschnitt (7) aufweist, dem ein Luftfilterelement (11) sowie ein Fluidabsperrglied (12) zugeordnet sind, **dadurch gekennzeichnet, dass** der zweite Kanalabschnitt (7) in dem Gehäuse (4) parallel zu dem ersten Kanalabschnitt ausgerichtet und zu einer Innenseite des Gehäuses hin offen ist, dass der offene Bereich des Kanalabschnitts (7) durch ein separat hergestelltes Verschlussteil (10) verschlossen ist, und dass der zweite Kanalabschnitt (7) einen Kanalausgang (9) zu der Außenseite des Gehäuses (4) aufweist, der parallel ausgerichtet ist zu dem ersten Kanalabschnitt (6) des Belüftungskanals (6, 7).

2. Einstechteil nach Anspruch 1, **dadurch gekennzeichnet, dass** das Verschlussteil eine 180°-Umlenkung zwischen dem ersten Kanalabschnitt (6) und dem zweiten Kanalabschnitt (7) bildet.

3. Einstechteil nach Anspruch 2, **dadurch gekennzeichnet, dass** das Verschlussteil als kraftschlüssig in einem Sitz des Gehäuses (4) positionierter Stopfen (10) gestaltet ist.

4. Einstechteil nach einem der vorhergehenden Ansprüche, **dadurch gekennzeichnet, dass** das Gehäuse (4) einschließlich des Einstechdorns (D) und der Kanalabschnitte (6, 7) des Belüftungskanals einteilig aus einem thermoplastischen Kunststoffmaterial in einem Spritzgussverfahren hergestellt ist.

5. Tropfkammer für ein medizinisches Infusionssystem, die mit einem Einstechteil (2) nach einem der vorhergehenden Ansprüche versehen ist.

## Claims

1. Piercing part for a medical infusion system, with a housing (4) which has a piercing mandrel (D), which housing has a fluid channel (5) and also a ventilation channel (6, 7), with a first channel portion (6) extending at least largely parallel to the fluid channel (5) and with a second channel portion (7) which is diverted towards an outside of the housing (4) and to which channel portion an air filter element (11) and a fluid shut-off member (12) are assigned, **characterized in that** the second channel portion (7) in the housing (4) is oriented parallel to the first channel portion and is open towards an inside of the housing, **in that** the open region of the channel portion (7) is closed by a separately produced closure part (10), and **in that** the second channel portion (7) has a channel outlet (9) to the outside of the housing (4), which channel outlet is oriented parallel to the first channel portion (6) of the ventilation channel (6, 7).

2. Piercing part according to claim 1, **characterized in that** the closure part forms a 180° diversion between the first channel portion (6) and the second channel portion (7).

3. Piercing part according to claim 2, **characterized in that** the closure part is designed as a stopper (10) positioned with force-fit engagement in a seat of the housing (4).

4. Piercing part according to any of the preceding claims, **characterized in that** the housing (4), including the piercing mandrel (D) and the channel portions (6, 7) of the ventilation channel, is produced in one piece from a thermoplastic material in an injection moulding process.

5. Drip chamber for a medical infusion system, provided with a piercing part (2) according to any of the preceding claims.

## Revendications

1. Pièce de perforation destinée à un système de perfusion médicale, ladite pièce de perforation comprenant un boîtier (4) qui est pourvu d'une pointe de perforation (D) et qui comporte un conduit de fluide (5) et un conduit de ventilation (6, 7), qui comporte une première portion de conduit (6) qui s'étend au moins dans une large mesure parallèlement au conduit de fluide (5) et une deuxième portion de conduit (7) qui est déviée vers un côté extérieur du boîtier (4) et à laquelle sont associés un élément formant filtre à air (11) et un organe d'arrêt de fluide (12), **caractérisée en ce que** la deuxième portion de conduit (7) dans le boîtier (4) est orientée parallèlement à la première portion de conduit et est ouverte en direction d'un côté intérieur du boîtier, **en ce que** la région ouverte de la portion de conduit (7) est fermée par une pièce de fermeture (10) fabriquée séparément et **en ce que** la deuxième portion de conduit (7) comporte une sortie de conduit (9) qui mène vers le côté extérieur du boîtier (4) et qui est orientée parallèlement à la première portion (6) du conduit de ventilation (6, 7).

2. Pièce de perforation selon la revendication 1, **caractérisée en ce que** la pièce de fermeture forme une déviation de 180° entre la première portion de conduit (6) et la deuxième portion de conduit (7).

3. Pièce de perforation selon la revendication 2, **caractérisée en ce que** la pièce de fermeture est conçue sous la forme d'un bouchon (10) positionné en force dans un siège du boîtier (4).

4. Pièce de perforation selon l'une des revendications précédentes, **caractérisée en ce que** le boîtier (4), y compris la pointe de perforation (D) et les portions (6, 7) du conduit de ventilation, est réalisé d'une seule pièce à partir d'une matière thermoplastique dans un procédé de moulage par injection.

5. Chambre de goutte à goutte destinée à un système de perfusion médical, laquelle chambre est munie d'une pièce de perforation (2) selon l'une des revendications précédentes.
